# EUROPEAN PATENT APPLICATION

(11) **EP 1 645 879 A1**
(43) Date of publication of application: **12.04.2006**
(21) Application number: 05021883.3
(22) Date of filing: 07.10.2005
(51) Int. Cl.: G01N 33/53

(54) **Screening system**

(30) Priority: 08.10.2004 JP 2004296437
(71) Applicant: FUJI PHOTO FILM CO., LTD., Minami-Ashigara-shi, Kanagawa-ken (JP)
(72) Inventor: Ogura, Nobuhiko, Ashigarakami-gun, Kanagawa-ken (JP)
(74) Representative: Klunker . Schmitt-Nilson . Hirsch

(57) **Abstract**

Bonding data regarding a great number of combinations of ligands and analytes are obtained within a short amount of time. Management of the bonding data and the corresponding analyte is automatically performed. A ligand solution (200) is supplied to a thin film layer of a measurement unit (12), prior to measurement by a measuring apparatus (11) that utilizes attenuated total reflection. The measuring unit (12) is held within a mounting space of a ligand immobilizing device (10), while a ligand in the ligand solution is being immobilized on the measuring unit. A specified analyte solution (300) is removed from an analyte storage section (302) of an analyte library (308), and supplied to the measuring apparatus (11) prior to measurement. Following measurement, analyte data regarding the specified analyte solution, and bonding data obtained by a bonding data obtaining section (331) are correlated with each other, recorded, and displayed.

## Description

### BACKGROUND OF THE INVENTION

### Field of the Invention

The present invention relates to a screening system for obtaining bonding data regarding combinations of pluralities of types of ligands and analytes, based on measurement results obtained by a measuring apparatus that utilizes attenuated total reflection. Particularly, the present invention relates to a screening system equipped with a ligand immobilizing device.

### Description of the Related Art

Screening is performed to find compounds, which may be candidates for pharmaceutical products, based on measurement results obtained by measuring apparatuses that utilize attenuated total reflection (as disclosed in Japanese Unexamined Patent Publication No. 2001-330560). The measurements obtain bonding data among a great number of different compounds (analytes) and a protein (ligand) of interest, which is a bioactive polymer substance. Examples of the bonding data are speeds of chemical bonding reactions, and speeds of chemical dissociating reactions. The compounds, which may be candidates for pharmaceutical products, are those that exhibit predetermined bonding data. Note that during screening, a ligand immobilizing process, in which a protein (ligand) is immobilized to a measurement cell utilized by the measuring apparatus, is necessary (as disclosed in Japanese Unexamined Patent Publication No. 6(1994)-167443).

In the system disclosed in Japanese Unexamined Patent Publication No. 6(1994)-167443, the ligand immobilizing process is performed in a state in which the measurement cell is placed in a measurement position. Therefore, measurement cannot be performed while the ligand immobilizing process is being administered. In systems of this type, there is a problem that an inordinate amount of time is necessary to obtain bonding data regarding a great number of combinations of analytes and ligands. In addition, along with the increase in the number of compounds (analytes) to be screened, it is becoming difficult to accurately manage correspondence among obtained bonding data and the analytes.

### SUMMARY OF THE INVENTION

The present invention has been developed in view of the foregoing circumstances. It is an object of the present invention to provide a screening system, which is capable of obtaining bonding data regarding a great number of combinations of ligands and analytes within a short amount of time. It is another object of the present invention to provide a screening system, in which management of the bonding data and the corresponding analyte is automatically performed.

The screening system of the present invention is a screening system, for performing measurements regarding combinations of pluralities of types of ligands and analytes, and for obtaining bonding data for each combination of ligands and analytes, comprising:
a measuring apparatus that utilizes attenuated total reflection, comprising:
   a measurement cell, constituted by: a dielectric block; a thin film layer, which is formed on a surface of the dielectric block and on which a ligand is immobilized; and an analyte holding mechanism, for holding an analyte on the thin film layer; and
   a measuring section, for causing a light beam to enter the dielectric block of the measurement cell, which is at a measurement position, at various angles of incidence, such that conditions for total internal reflection are obtained at an interface between the dielectric block and the thin film layer, and for measuring the state of attenuated total reflection of the light beam, which is totally internally reflected at the interface; and
bonding data obtaining means, for obtaining bonding data regarding the ligand and the analyte, based on the measurement results obtained by the measuring apparatus; characterized by further comprising:
   ligand immobilizing means, for supplying a ligand to the thin film layer of the measurement cell at a position different from the measurement position prior to the measuring apparatus performing measurement, and for holding the thin film layer while the ligand is being immobilized on the thin film layer;
   an analyte library, equipped with an analyte database for recording storage sections, at which each of the plurality of types of analytes is stored, correlated with analyte data for each type of analyte;
   analyte supplying means, for removing a specified analyte from an analyte storage section and for supplying the specified analyte to the measuring apparatus;
   memory means for recording the analyte data of the specified analyte, correlated with the bonding data obtained by the bonding data obtaining means; and
   display means, for displaying the analyte data of the specified analyte read out from the memory means, correlated with the bonding data obtained by the bonding data obtaining means.

Note that the measurement cell may be that in which the dielectric block, the thin film layer, and the analyte holding mechanism are formed integrally, or each of the components may be separate parts. Alternatively, the dielectric block may be constructed as a separate part, and the thin film layer and the analyte holding mechanism may be integrally formed, or the dielectric block and the thin film layer may be integrally formed, and the analyte holding mechanism may be constructed as a separate part. Note that during immobilization of ligands, the immobilizing position may be provided at the portion of the thin film layer, which is formed integrally with the dielectric block.

Note that here, the "ligand" is a compound, which is immobilized on the thin film layer. Preferably, the "ligand" is a bioactive polymer substance. Also, the "analyte" is a compound that possibly bonds with the "ligand".

Note that the "compound" is that which is structured by molecules constituting at least two elements. In the present invention, the term "compound" applies mainly to those that have molecular weights within a range of 50 to 1, 000, 000. However, the term "compound" may apply to substances having molecular weights less than or greater than the above range. Bioactive polymer substances include at least one of the sugars, amino acids, nucleotides, fats, hemes, quinones, proteins, RNA, DNA, phosphatides, polysaccharides, nucleic acids, and enzymes listed in "Natural Polymers", Japanese Industrial Standards Document No. K3611, symbols 11001-11025; "1. Introduction to Biological Materials", Encyclopedia of Biological Data, Asakura Press; and "II. Biological Materials and Metabolism", Bioscience Dictionary, Asakura Press.

Here, "bonding data" refers to data that reflects states of bonding. Examples of such data are: sensorgrams that graph the change in attenuated total reflection angles over time; bonding speed constants ka; dissociating speed constants kd; dissociating constants KD; and maximum bonding amounts Rmax. The "bonding data" may be judgment results regarding whether the values of the bonding speed constants ka; the dissociating speed constants kd; the dissociating constants KD; and the maximum bonding amounts Rmax are within a predetermined range. Note that the bonding speed constant ka is a value that indicates the speed of a bonding reaction, and the dissociating speed constant kd is a value that indicates the speed of a dissociating reaction. The dissociating constant KD is a value derived by dividing the dissociating speed constant kd by the bonding speed constant ka. That is, the dissociating constant KD can be expressed by the formula: Dissociating Constant KD = dissociating speed constant kd /bonding speed constant ka. Further, the maximum bonding amount Rmax indicates the amount of chemical bonding between the analyte (sample) and the bioactive polymer substance, when the chemical bonding reaction between the analyte and the bioactive polymer substance reaches a state of saturation.

"Analyte data" may be: the name of an analyte; a molecular weight of the analyte; a buffer concentration; bonding data obtained by prior measurement; and the like. Ligand data may be: the name of a ligand; immobilization protocols, such as immobilization temperature or immobilization time; and the like.

The bonding data obtaining means may obtain the bonding data regarding the ligand and the analyte, based on the measurement results of the measuring apparatus and the analyte data.

The analyte database may record the bonding data regarding the ligand and the analyte obtained by the bonding data obtaining means, correlated with the analyte data of the analyte.

A configuration may be adopted, wherein:
the analyte supplying means comprises: a plurality of solution housing plates, which have ID's, and in which a plurality of solution housing portions are arranged two dimensionally; and analyte dispensing means, for removing a specified analyte from the analyte storage section and supplying the specified analyte to a solution housing portion of a solution housing plate; and
the memory means records the ID of the solution housing plate, the position of the solution housing portion, and the analyte data regarding the specified analyte, correlated to each other.

A configuration may be adopted, wherein the screening system further comprises:
a ligand library, equipped with a ligand database for recording storage sections, at which each of the plurality of types of ligands is stored, correlated with ligand data for each type of ligand; and
ligand supplying means, for removing a specified ligand from a ligand storage section and for supplying the specified ligand to the ligand immobilizing means; wherein:
   the memory means records the ligand data of the specified ligand, correlated with the bonding data obtained by the bonding data obtaining means; and
   the display means displays the ligand data of the specified ligand read out from the memory means, correlated with the bonding data obtained by the bonding data obtaining means.

A configuration may be adopted, wherein:
the ligand supplying means comprises: a plurality of solution housing plates, which have ID's, and in which a plurality of solution housing portions are arranged two dimensionally; and ligand dispensing means, for removing a specified ligand from the ligand storage section and supplying the specified ligand to a solution housing portion of a solution housing plate; and
the memory means records the ID of the solution housing plate, the position of the solution housing portion, and the ligand data regarding the specified ligand, correlated to each other.

Note that "arranged two dimensionally" refers to an arrangement, in which at least two solution housing portions are provided in both rows and columns.

Measurements that utilize attenuated total reflection are those that utilize the fact that attenuated total reflection angles change, according to dielectric constants in the vicinity of a metal surface. Attenuated total reflection angles are specific reflective angles that cause sudden attenuation (attenuated total reflection) in light intensity of a light beam, which is totally internally reflected at a surface of metal, due to generation of surface plasmon. Surface plasmon measuring apparatuses are known as measuring apparatuses that utilize attenuated total reflection. As another type of sensor that utilizes attenuated total reflection, there is known a leaky mode sensor as described in, for instance, "Surface Refracto-Sensor using Evanescent Waves: Principles and Instrumentations" by Takayuki Okamoto, Spectrum Researches, Vol. 47, No.1 (1998), pp21-23 &pp26-27. The leaky mode sensor basically comprises: a dielectric block, shaped as a prism, for example; a cladding layer, formed on one surface of the dielectric block; an optical waveguide layer, which is formed on the cladding layer and which is brought into contact with a sample; a light source for emitting a light beam; an optical system for causing the light beam to enter the dielectric block at various angles of incidence so that total internal reflection conditions are satisfied at an interface of the dielectric block and the cladding layer; a photodetecting means for measuring the intensity of the light beam, which has been totally reflected at the interface, to measure the state of excitation of a waveguide mode, that is, the state of attenuated total reflection.

In the screening system of the present invention, a ligand is supplied to the thin film layer of the measurement cell, which is at a position different from the measurement position, prior to the measuring apparatus performing measurement, and the thin film layer is held while the ligand is being immobilized on the thin film layer. Therefore, measurement and the ligand immobilizing process can be performed in parallel, improving efficiency in immobilization of ligands and obtainment of bonding data. Accordingly, bonding data regarding a great number of combinations of ligands and analytes are enabled to be obtained within a short period of time. Further, the screening system comprises the analyte library, equipped with the analyte database for recording storage sections, at which each of the plurality of types of analytes is stored, correlated with analyte data for each type of analyte. A specified analyte is removed from an analyte storage section and supplied to the measuring apparatus prior to measurement. After measurement, the analyte data of the specified analyte is recorded and displayed, correlated with the bonding data obtained by the bonding data obtaining means. Thereby, the correspondence among obtained bonding data and analytes can be managed automatically.

The bonding data obtaining means may obtain the bonding data regarding the ligand and the analyte, based on the measurement results of the measuring apparatus and the analyte data. In this case, more accurate bonding data is enabled to be obtained.

The analyte database may record the bonding data regarding the ligand and the analyte obtained by the bonding data obtaining means, correlated with the analyte data of the analyte. If this configuration is adopted, the bonding data may be employed as a candidate selecting condition, in the case that focused screening, in which the number of analytes to be screened is narrowed by certain conditions, is to be performed. Thereby, the hit rate of the analytes can be improved.

A configuration may be adopted, wherein: the analyte supplying means comprises: the plurality of solution housing plates, which have ID' s, and in which the plurality of solution housing portions are arranged two dimensionally; and the analyte dispensing means, for removing a specified analyte from the analyte storage section and supplying the specified analyte to a solution housing portion of a solution housing plate; and wherein the memory means records the ID of the solution housing plate, the position of the solution housing portion, and the analyte data regarding the specified analyte, correlated to each other. In this case, erroneously selecting an analyte, which is not the specified analyte, can be prevented, thereby the reliability of screening can be improved.

A configuration may be adopted, wherein the screening system further comprises: the ligand library, equipped with the ligand database for recording storage sections, at which each of the plurality of types of ligands is stored, correlated with ligand data for each type of ligand; and the ligand supplying means, for removing a specified ligand from a ligand storage section and for supplying the specified ligand to the ligand immobilizing means; wherein: the memory means records the ligand data of the specified ligand, correlated with the bonding data obtained by the bonding data obtaining means; and the display means displays the ligand data of the specified ligand read out from the memory means, correlated with the bonding data obtained by the bonding data obtaining means. In this case, the correspondence among the obtained bonding data and ligands can be managed automatically.

A configuration may be adopted, wherein: the ligand supplying means comprises: the plurality of solution housing plates, which have ID' s, and in which the plurality of solution housing portions are arranged two dimensionally; and the ligand dispensing means, for removing a specified ligand from the ligand storage section and supplying the specified ligand to a solution housing portion of a solution housing plate; and wherein the memory means records the ID of the solution housing plate, the position of the solution housing portion, and the ligand data regarding the specified ligand, correlated to each other. In this case, erroneously selecting a ligand, which is not the specified ligand, can be prevented, thereby the reliability of screening can be improved.

### BRIEF DESCRIPTION OF THE DRAWINGS

Figure 1 is a schematic structural view of a screening system according to an embodiment of the present invention.
Figure 2 is a diagram for explaining a ligand immobilizing method.
Figure 3 is a diagram for explaining a surface plasmon measuring method.
Figure 4 is an exploded perspective view of a measuring unit.
Figure 5 is a perspective schematic view of a ligand immobilizing device.
Figure 6 is a block diagram illustrating the construction of a temperature adjusting section.
Figure 7 is a structural diagram of a measuring apparatus.
Figure 8 illustrates bonding data, which is displayed at a display section.

### DESCRIPTION OF THE PREFERRED EMBODIMENTS

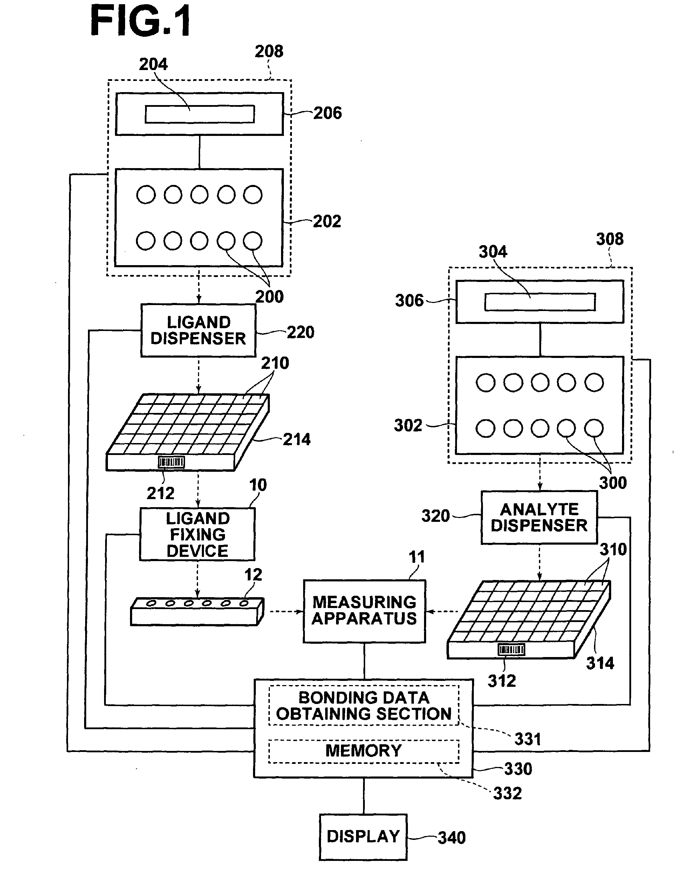

Hereinafter, an embodiment of the present invention will be described with reference to the drawings. Figure 1 is a schematic structural view of a screening system according to the embodiment of the present invention. The screening system comprises: a measuring apparatus 11, for measuring states of attenuated total reflection that reflects bonding states of ligands and analytes; a ligand storage section 202, at where a great number of types of ligand solutions 200 are stored; a ligand library 208 constituted by a ligand database 204, in which ligand data 204 regarding each ligand solution 200 stored in the ligand storage section 202 is recorded; a plurality of solution housing plates 214, in each of which a plurality of solution housing portions 210 are formed, and on each of which bar codes 212 are attached; a ligand dispenser 220, for dispensing ligand solutions 200 from the ligand storage section 202 to the solution housing portions 210 of the solution housing plates 214; a ligand immobilizing device 10, for immobilizing ligands within the ligand solutions 200 onto sensor surfaces 13a of measurement cells 17, which are provided in a measuring unit 12; an analyte storage section 302, at where a great number of types of analyte solutions 300 are stored; an analyte library 308 constituted by an analyte database 304, in which analyte data 304 regarding each analyte solution 300 stored in the analyte storage section 302 is recorded; a plurality of solution housing plates 314, in each of which a plurality of solution housing portions 310 are formed, and on each of which bar codes 312 are attached; an analyte dispenser 320, for dispensing analyte solutions 300 from the analyte storage section 302 to the solution housing portions 310 of the solution housing plates 314; a control section 330, which is connected to the measuring apparatus 11, the ligand library 208, the ligand dispenser 220, the ligand immobilizing device 10, the analyte library 308, and the analyte dispenser 320, for obtaining bonding data regarding the ligands and analytes, based on measurement results output from the measuring apparatus 11, and for controlling the operations of each of the components; and a display section 340, which is connected to the control section 330.

The screening system of the present invention screens analyte solutions that contain analytes that bond with ligands, from among the analyte solutions stored in the analyte library 308. Whether the analytes bond with the ligands is judged, based on measurement results, obtained by measuring states of attenuated total reflection that reflect bonding states among the analytes and the ligands. In order to measure the states of attenuated total reflection of the ligands and analytes, first, a great number of measurement cells 17, on which ligands have been immobilized. Then, different types of analyte solutions are added to each of the measurement cells 17, light beams are irradiated into each measurement cell 17, and the states of attenuated total reflection of the totally internally reflected light beams are measured.

In order to simplify the description, first, a ligand immobilizing method and an attenuated total reflection measuring method for a single measurement cell 17 will be described. Thereafter, the construction and operations of the screening system of the present invention will be described.

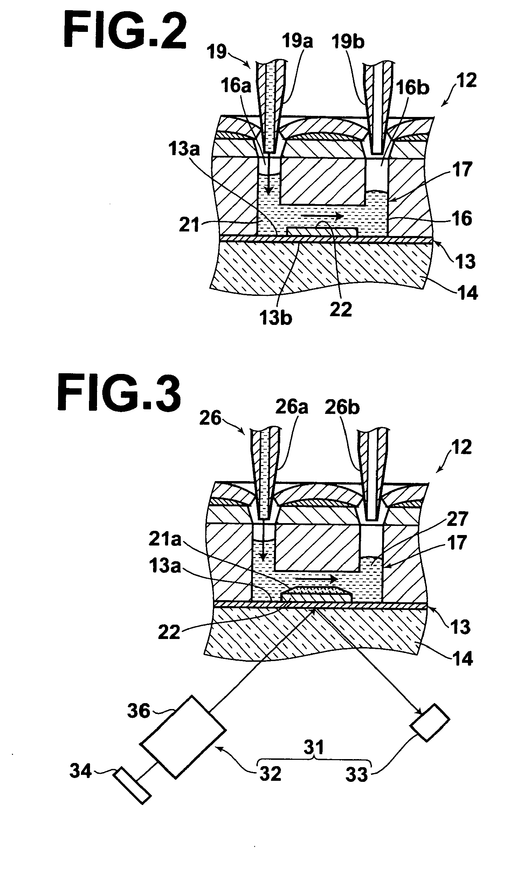

First, the measuring cell 17 will be described. As illustrated in Figure 2, the measuring cell 17 comprises: a metal film 13, which becomes the sensor surface 13a where surface plasmon is generated, provided on one surface; a prism 14, which is attached to a light incident surface 13b opposite the sensor surface 13a; and a flow path 16, which is provided to face the sensor surface 13a and through which ligand solutions and analyte solutions are caused to flow.

Gold, for example, is used as the material of the metal film 13. The film thickness of the metal film 13 is 500 angstroms, for example. The film thickness is appropriately selected according to the material of the metal film, the wavelength of light to be irradiated thereon, and the like. The prism 14 is a transparent dielectric, on the upper surface of which the metal film 13 is formed. The prism 14 condenses irradiated light toward the light incident surface 13b such that conditions for total internal reflection are satisfied. The flow path 16 is substantially in the form of a U-shape, and comprises an entrance 16a, through which liquids are supplied, and an exit 16b, through which liquids are discharged. The diameter of the flow path 16 is 1mm, for example, and the interval between the entrance 16a and the exit 16b is 10mm, for example.

The bottom portion of the flow path 16 is open, and the open portion is sealed by the sensor surface 13a. The measurement cell 17 is constituted by flow paths 16, constructed as described above, and the sensor surface 13a. As will be described later, the measuring unit 12 comprises a plurality of measurement cells 17.

Hereinafter, the method for immobilizing ligands will be described. Ligands are immobilized to the sensor surface 13a of the measurement cell 17. First, the measuring unit 12, that is, the measurement cell 17, is set in the ligand immobilizing device 10, which will be described later. The ligand immobilizing device comprises a pipette pair 19, constituted by a pipette 19a and a pipette 19b. The pipette 19a and the pipette 19b are inserted into the entrance 16a and the exit 16b, respectively. The pipette 19a functions to supply liquid into the flow path 16, and the pipette 19b functions to suction the liquid from the flow path 16. They operate in a linked manner, such that during supply of liquid by the pipette 19a, suction of liquid is performed by the pipette 19b. The pipette pair 19 is employed to supply a ligand solution 200, in which a ligand is dissolved, through the entrance 16a.

A linker film 22 is formed at the approximate center of the sensor surface 13a. The linker film 22 is formed in advance, during a manufacturing step of the measuring unit 12. The linker film 22 becomes an immobilizing base for the ligand, and therefore is appropriately selected according to the type of ligand.

Prior to performing the ligand immobilizing process, in which the ligand solution 200 is supplied, first, an immobilizing buffer solution is supplied to moisten the linker film. Next, an activation process is administered to the linker film 22, so as to facilitate bonding of the ligand with the linker film 22. In an amine coupling method, for example, carboxymethyl dextran is utilized as the linker film, to cause amino bases within the ligand to directly covalently bond with the dextran. In this case, a mixture of 1-Ethyl-3-[3-dimethylaminopropyl] carbodiimide hydrochloride (EDC) and N-hydroxy succinimide (NHS) is utilized as an activating solution. After the activation process, the flow path 16 is cleansed with an immobilizing buffer.

Various types of buffer solutions, organic saline solutions, and distilled water may be utilized as the immobilizing buffer and the solution (diluent) of the ligand solution 200.

The types of solutions, their pH values, the types of components therein, their concentrations, and the like are determined according to the type of the ligand. For example, in the case that biological material is utilized as the ligand, it is often the case that physiological saline solution having an adjusted neutral pH value is employed as the solution. However, in the aforementioned anime coupling method, the linker film 22 is negatively charged by the carboxymethyl dextran. Therefore, phosphatic buffered saline (PBS), which contains phosphate at a higher concentration than physiological saline, may be used to positively charge proteins, in order to facilitate bonding with the negatively charged linker film 22.

After the activation process and cleansing are performed, the ligand solution 200 is supplied to the measurement cell 17, and the ligand immobilizing process is initiated. When the ligand solution 200 is supplied to the flow path 16, a ligand 21a, which is dispersed within the ligand solution 200, gradually approach the linker film 22 and bond therewith. In this manner, the ligand 21a is immobilized to the sensor surface 13a. Immobilization generally occurs after approximately one hour. During this time, the measuring unit 12 is stored in a state in which environmental conditions, such as temperature, are set at predetermined levels. Note that the ligand solution 200 wihtin the flow path 16 may be kept static during immobilization, but it is preferable for the ligand solution 200 to be agitated and caused to flow within the flow path 16. Agitation of the ligand solution 200 accelerates bonding between the ligand 21a and the linker film 22, and the amount of the ligand 21a immobilized on the linker film 22 can be increased.

When immobilization of the ligand 21a onto the sensor surface 13a is completed, the ligand solution 200 is discharged from the flow path 16, by being suctioned by the pipette 19b. The sensor surface 13a, on which the ligand 21a has been immobilized, is subjected to a cleansing operation, by cleansing liquid being supplied to the flow path 16. After the cleansing operation, a blocking process is performed as necessary, to deactivate reactive bases of the linker film 22 at portions thereof at which the ligand 21a did not bond. The blocking process is performed by supplying a blocking liquid, such as ethanol amine-hydrochloride, to the flow path 16. The flow path 16 is cleansed again after the blocking process. After this cleansing operation, the measuring unit 12 is conveyed to the measuring apparatus 11.

Next, the method of measuring the state of attenuated total reflection will be described with reference to Figure 3. Measurement of the state of attenuated total reflection is performed while the measuring unit 12 is set in the measuring apparatus 11. A pipette pair 26, similar to the pipette pair 19 of the ligand immobilizing device, is provided in the measuring apparatus 11. Various liquids are supplied to the flow path 16 from the pipette pair 26, through the entrance 16a. First, a measuring buffer liquid is supplied to the flow path 16.

Next, an analyte solution 300, in which an analyte is dissolved, is supplied to the flow path 16, and the measuring buffer liquid is supplied again thereafter. Note that the flow path 16 may be cleansed prior to the first supply of the buffer liquid. Measurement of the state of attenuated total reflection is initiated immediately after the buffer liquid is supplied the first time, in order to obtain a reference signal level. Measurement is continued through supply of the analyte solution 300, the second supply of the buffer liquid, and for a predetermined amount of time thereafter. Thereby, the reference signal level can be obtained, and states of attenuated total reflection that reflect the bonding state of the analyte and the ligand and the dissociating state of the analyte and the ligand, due to the supply of the measuring buffer liquid, can be observed.

Although not illustrated in the figures, a reactive region (act) and a reference region (ref) are formed on the linker film 22. The ligand is immobilized in the reactive region, and the reaction between the analyte and the ligand occurs therein. The ligand is not immobilized in the reference region, which is provided such that a reference signal can be obtained during measurement of signals within the reactive region. The reference region is formed when the linker film 22 is formed. Administering a surface process on the linker film 22 to deactivate bonding bases within a region, which is approximately half the area of the linker film 22, is an example of a process for forming the reference region. By this process, half of the linker film 22 becomes the reactive region, and the remaining half becomes the reference region.

Signals regarding the reactive region (act signals) and signals regarding the reference region (ref signals) are measured simultaneously from the detection of the reference signal level, through the bonding reaction and dissociation. The bonding data is obtained by deriving differences and ratios of the act signals and the ref signals. The control section 330 obtains a difference between the act signals and the ref signals, and designates the difference as measurement data, based on which the bonding data is obtained, for example. By adopting this configuration, noise caused by external factors, such as: differences in individual measuring units or measuring cells; mechanical displacement of the apparatus; temperature change of the liquid; and the like can be canceled, thereby yielding signals with favorable S/N ratios.

Various types of buffer solutions, organic saline solutions, and distilled water may be utilized as the immobilizing buffer and the solution (diluent) of the analyte solution 300. The types of solutions, their pH values, the types of components therein, their concentrations, and the like are determined according to the type of the analyte. For example, dimethyl sulfoxide (DMSO) may be mixed into physiological saine, in order to facilitate dissolution of analytes therein. Note that DMSO greatly affects the state of attenuated total reflection. As described previously, the measuring buffer liquid is employed to detect a reference signal level. Therefore, in the case that DMSO is mixed into the analyte solution, it is preferable for the measuring buffer liquid to have a DMSO concentration similar to that of the analyte solution.

As illustrated in Figure 3, a measuring section 31 comprises an illuminating section 32 and a detector 33. As described previously, states of the reaction between the ligand and the analyte appears as changes in attenuated total reflection angels. Therefore, the illuminating section 32 emits light such that the light enters the light incident surface 13b at various angles of incidence that satisfy total internal reflection conditions. The illuminating section 32 comprises: a light source 34; and an optical system 36 constituted by a condensing lens, a diffusion plate, and a polarizing plate, for example. The illuminating section 32 is provided at a position and angle such that the emitted light satisfies the aforementioned conditions for total internal reflection.

A light emitting element, such as an LED (Light Emitting Diode), an LD (Laser Diode), an SLD (Super Luminescent Diode), or the like is utilized as the light source 34. A single light emitting element is utilized to emit light toward a single measurement cell 17. Note that in the case that a plurality of measurement cells 17 are to be measured simultaneously, light emitted from a single light source may be separated so as to irradiate the plurality of measurement cells. Alternatively, a plurality of light emitting elements may be provided and arranged to correspond to each of the plurality of measurement cells. The diffusion plate diffuses the light from the light source 34, to suppress fluctuations in the amount of light emitted by a light emitting surface thereof. The polarizing plate only transmits p-polarized light that causes surface plasmon to be generated, from the emitted light. Note that the polarizing plate is not necessary, if the light beam emitted by the light source has an aligned polarization direction, such as in the case that an LD is employed. However, even if the light source emits light having an aligned polarization direction, in the case that the polarization direction becomes misaligned due to the emitted light being diffused by the diffusion plate, the polarizing plate may be employed to realign the polarization direction. The light, which is diffused and polarized in this manner, is focused by the condensing lens, and is irradiated onto the prism 14. Thereby, a light beam having various angles of incidence and no fluctuations in the amount of light thereof, is caused to enter the light incident surface 13b.

The detector 33 receives the light reflected at the light incident surface 13b, and detects the light intensity thereof. Light beams enter the light incident surface 13b at various angles. Therefore, the light beams are reflected at various reflective angles corresponding to the incident angles. If a resonance angle changes according to a reactive state between the analyte and the ligand, the attenuated total reflection angle, at which the light intensity becomes attenuated, also changes. A CCD area sensor is utilized as the detector 33, for example. The detector 33 detects changes in the attenuated total reflection angle, by detecting movement of an attenuated position of the reflected light within its light receiving surface. The detector 33 outputs measurement data regarding the attenuated total reflection angle, obtained in this manner, to the control section 330. The control section 330 obtains bonding data regarding the ligand and the analyte based on the measurement data obtained by the measuring apparatus 11, and displays the bonding data at the display section 340. Note that the details of the operations of the control section 330 will be described later.

In order to clarify the construction of the measuring section 31, the illuminating section 32 and the detector 33 are illustrated in Figure 3 in an arrangement such that the direction of the light beam incident on the light incident surface 13b and the light beam reflected therefrom are parallel to the direction that the liquid flows within the flow path 16. However, in the present embodiment, the illuminating section 32 and the detector 33 are provided such that the direction of the incident light beam and the reflected light beam are perpendicular to the direction that the liquid flows. However, the measuring section 31 may be provided as illustrated in Figure 3, and measurement may be performed with this configuration as well.

Next, the construction and the operation of the screening system according to the present embodiment will be described. As described previously, the screening system comprises: the measuring apparatus 11; the ligand library 208; the ligand solution housing plates 214, in which the great number of ligand housing portions 210 are formed; the ligand dispenser 220 for dispensing ligand solutions 200 from the ligand storage section 202 to the solution housing portions 210 of the solution housing plates 214; the ligand immobilizing device 10, for immobilizing ligands within the ligand solutions 200 onto the sensor surfaces 13a of measurement cells 17, which are provided in a measuring unit 12; the analyte library 308; the plurality of analyte solution housing plates 314, in each of which the plurality of analyte solution housing portions 310 are formed; the analyte dispenser 320, for dispensing analyte solutions 300 from the analyte storage section 302 to the analyte solution housing portions 310 of the analyte solution housing plates 314; the control section 330, which is connected to the measuring apparatus 11, the ligand library 208, the ligand dispenser 220, the ligand immobilizing device 10, the analyte library 308, and the analyte dispenser 320; and the display section 340, which is connected to the control section 330.

The ligand library 208 comprises: the ligand storage section 202, at where the great number of ligand solutions 200 that contain proteins as ligands are stored; and the ligand database 204, in which ligand data 204 regarding each ligand solution 200 stored in the ligand storage section 202 is recorded. The names of the proteins and immobilization protocols, such as immobilization temperatures, immobilization times, and the like may be recorded as the ligand data 204.

The analyte library 308 comprises: the analyte storage section 302, at where the great number of analyte solutions 300 that contain compounds as analytes are stored; and the analyte database 304, in which analyte data 304 regarding each analyte solution 300 stored in the analyte storage section 302 is recorded. The names of the compounds, the molecular weights thereof, buffer concentrations, and bonding data regarding the compounds and proteins are recorded as the analyte data 304. Here, "buffer concentrations" refer to, for example, DMSO (dimethyl sulfoxide) concentration, in the case that the solution used to dissolve the analyte is physiological saline and DMSO. In addition, "bonding data regarding the compounds and proteins" refers to bonding data regarding the analytes and proteins, obtained in a previous screening operation.

The ligand solution housing plates 214 have 48 solution housing portions 210 formed therein, in six rows and eight columns. The bar codes 212 are attached on the side surfaces of the ligand solution housing plates 214. The analyte solution housing plates 314 also have 48 solution housing portions 310 formed therein, in six rows and eight columns. The bar codes 312 are attached on the side surfaces of the analyte solution housing plates 314.

The ligand dispenser 220 supplies a ligand solution 200, which is stored at a specific location in the ligand storage section 202, to a specific solution housing portion 210 of a ligand solution housing plate 214, according to control from the control section 340. The ligand dispenser 220 has a bar code reading function, for reading out the data recorded in the bar codes 212, which are attached to the side surfaces of the ligand solution housing plates 214.

The analyte dispenser 320 supplies an analyte solution 300, which is stored at a specific location in the analyte storage section 302, to a specific solution housing portion 310 of a ligand solution housing plate 314, according to control from the control section 340. The analyte dispenser 320 has a bar code reading function, for reading out the data recorded in the bar codes 312, which are attached to the side surfaces of the analyte solution housing plates 314.

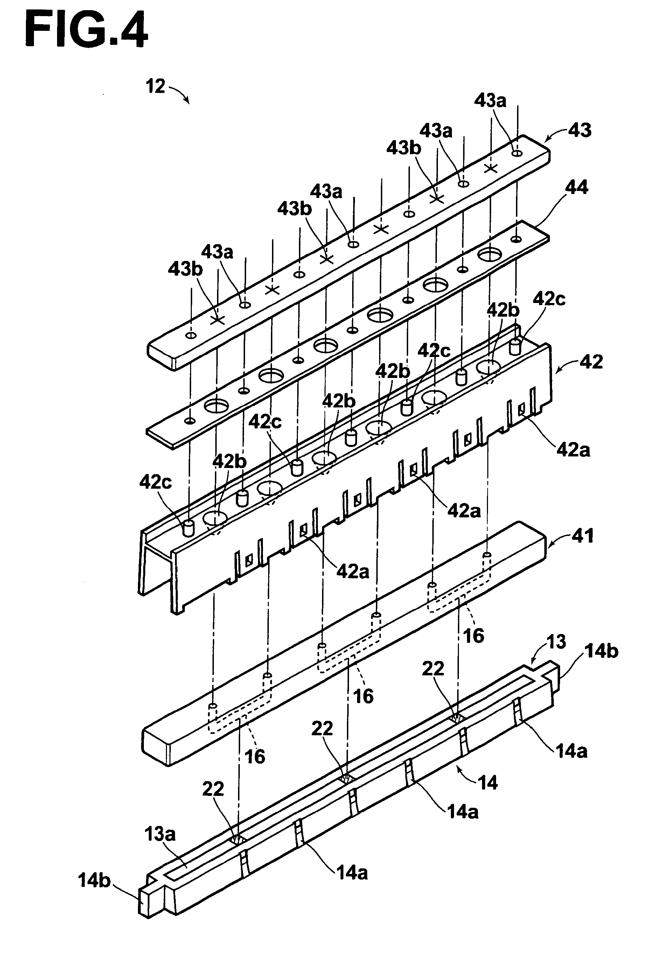

Each of the measuring units 12 are constituted by three measurement cells 17, integrally formed with each other. Figure 4 is an exploded perspective view of a measuring unit 12. The measuring unit 12 comprises: a flow path member 41 that forms flow paths 16; the prism 14, on the upper surface of which the metal film 13 is formed; a holding member 42, for holding the flow path member 41 in a state in which the bottom surface thereof is in contact with the upper surface of the prism 14; and a lid member 43, which is provided above the holding member 42.

Three flow paths 16 are formed in the flow path member 41. The flow path member 41 is of an elongate shape, and the three flow paths 16 are formed along the longitudinal direction thereof. The flow paths 16, with the metal film 13 that contacts the bottom surfaces thereof, constitute the measurement cells 17. For this reason, the flow path member 41 is formed by an elastic material, such as rubber or PDMS (polydimethyl siloxane), in order to improve the close contact properties with the metal film 13. Thereby, when the bottom surface of the flow path member 41 is pressed against the upper surface of the prism 14, the flow path member 41 deforms elastically, to eliminate gaps in the contact surface with the metal film 13. Accordingly, the open bottoms of the flow paths 16 are surrounded in a watertight manner atop the upper surface of the prism 14. Note that in the present example, a description is given regarding a measuring unit 12 comprising three flow paths 16. However, the number of flow paths 16 is not limited to being three, and may be one, two, or four or more.

The metal film 13 is formed on the upper surface of the prism 14 by vapor deposition. The metal film 13 is formed as a strip that faces the plurality of flow paths 16 formed in the flow path member 41. The linker films 22 are formed on the upper surface (sensor surface 13a) of the metal film 13, at portions that face each of the flow paths 16. Engaging claws 14a, for engaging with engaging portions 42a of the holding member 42, are formed along both side surfaces of the prism 14. The flow path member 41 is sandwiched between the holding member 42 and the prism 14 by the engagement between the engaging claws 14a and the engaging portions 42a, and held in a state in which the bottom surface thereof is pressed in contact with the upper surface of the prism 14. The flow path member 41, the metal film 13 and the prism 14 are integrated in this manner.

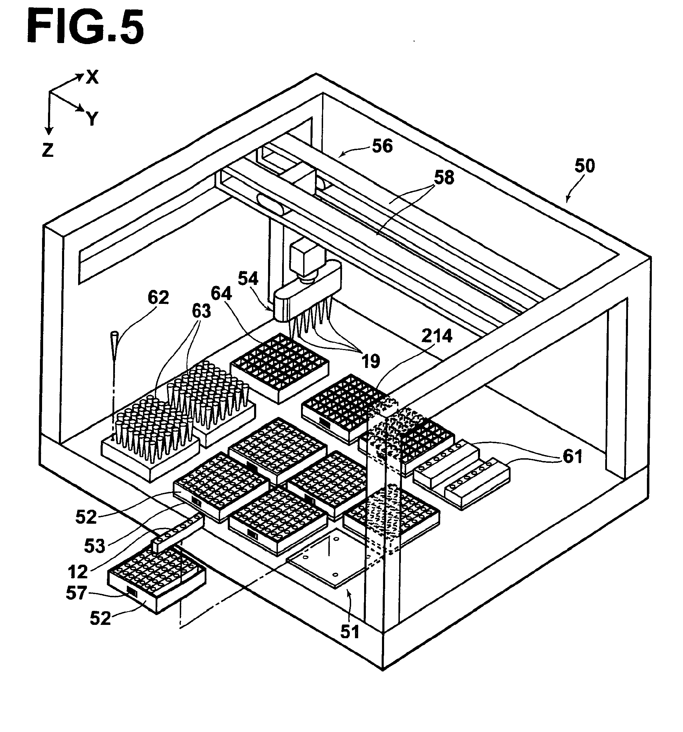

Protrusions 14b are provided at both ends in the longitudinal direction of the prism 14. As will be described later, the measuring unit 12 is immobilized in a state in which it is held in a holder 52 (refer to Figure 5). The protrusions 14b engage with the holder 52 to position the measuring unit 12 within the holder at a predetermined position.

Receiving openings 42b that the tips of the pipettes (19a, 19b, 26a, 26b) enter through are provided in the upper portion of the holding member 42, at positions that correspond to the entrance 16a and the exit 16b of each flow path 16. The receiving openings 42b are formed as funnels, such that the liquids expelled from the pipettes are led to the entrances 16a. When the holding member 42 engages with the prism 14 to sandwich the flow path member 41 therebetween, the lower surfaces of the receiving openings 42b contact the entrances 16a and the exits 16b. Thereby, the receiving openings 42b become linked with the flow paths 16.

Cylindrical bosses 42c are formed on both sides of each of the receiving openings 42b. The bosses 42c engage with holes 43a, which are formed in the lid member 43, to position the lid member 43. The lid member 43 is adhesively attached to the upper surface of the holding member 42 with double sided adhesive tape, which has openings at positions that correspond to the receiving openings 42b and the holes 43a.

The lid member 43 prevents evaporation of liquids within the flow paths 16, by covering the receiving openings 42b that communicate with the flow paths 16. The lid member 43 is formed by an elastic material, such as rubber or plastic, and cruciform slits 43b are formed at positions that correspond to each of the receiving openings 42b. Because the lid member 43 is provided to prevent evaporation of liquids within the flow paths 16, it is necessary for the lid member 43 to cover the receiving openings 42b. However, if the receiving openings 42b are completely sealed, it will not be possible to insert the pipettes therethrough. Therefore, the slits 43b are formed to enable insertion of the pipettes, and to seal the receiving openings 42b in a state in which the pipettes are not inserted. When the pipettes are inserted, the peripheries of the slits 43b elastically deform (refer to Figure 2) to form openings that receive the pipettes. When the pipettes are extracted, the slits 43b return to their initial state due to elastic force, and the receiving openings 42b are sealed thereby.

As illustrated in Figure 5, the ligand immobilizing device 10 comprises: a frame base 50; and a placement space 51, formed within the frame base 50, that serve as immobilization positions for a plurality of measuring units 12 placed thereon. All of the steps of the immobilization process are administered to the measuring units 12 while the measuring units 12 are placed on the placement space 51. Accordingly, the placement space 51, at which the immobilization steps are administered, is a immobilization stages for the measuring units 12.

The measuring units 12 are set in the ligand immobilizing device 10 in a state in which they are housed inside the holder 52. The holder 52 is configured to house a plurality (for example, eight) of the measuring units 12. Engaging portions, for engaging with the protrusions 14b of the measuring units 12, are provided in the holder 52. The bottom portion of the holder 52 open, except for supporting portions that support the ends of the measuring units 12. As will be described later, in the case that the measuring units 12 are removed from the holder 52 during a measuring process, a thrusting member (not shown) is inserted through the open bottom, to thrust the measuring unites 12 upward. A bar code 57 is attached to each holder 52.

The placement space 51 is configured to be able to accommodate ten holders 52, for example. A number of bases 53, corresponding to the number of holders 52 to be placed, are provided in the placement space 52. Positioning bosses, for positioning the holders 52, are provided on each of the bases 53.

A pipette head 54, constituted by three pipette pairs 19 are mounted on a head main body, is provided in the ligand immobilizing device 10. The pipette head 54 accesses the measuring units 12, which are on a conveyor belt (not shown) provided on the placement space 51, to supply and discharge liquids. Three pipette pairs 19 are provided on the pipette head 54. Therefore, simultaneous supply (and discharge) of liquids to the three measurement cells 17 of a single measuring unit 12 is enabled. A controller (not shown) is provided in the ligand immobilizing device 10. Suctioning and discharging operations, such as: timings, amounts to be suctioned and amounts to be discharged, for each pipette pair 19 of the pipette head 54 are controlled by the controller.

A head moving mechanism 56, for moving the pipette head 54 in the X, Y, and Z directions, is provided in the frame base 50. The head moving mechanism 56 is a known moving mechanism constituted by: a conveyor belt, pulleys, a carriage, and a motor, for example. The head moving mechanism 56 comprises: an elevating mechanism for moving the pipette head 54 vertically; a Y direction moving mechanism, including guide rails 58 that hold the pipette head 54 such that it is movable in the Y direction; and an X direction moving mechanism, for holding the guide rails 58 at both ends thereof and for moving the pipette head 54 in the X direction, along with the guide rails 58. The head moving mechanism 56 is controlled by the controller. The controller controls the position of the pipette head 54 in the vertical and horizontal directions, by driving the head moving mechanism 56.

The ligand solution housing plates 214 that house the ligand solutions 200 therein, and a plurality of liquid storage sections 61 that store various liquids (cleansing liquid, immobilization buffers, activating liquid, blocking liquid, and the like) are provided above the frame base 50. The number of liquid storage sections 61 is determined according to the number of types of liquids to be utilized. Six insertion openings are provided in each of the liquid storage sections 61. The number and arrangement pitch of the insertion openings are determined according to the number and arrangement pitch of the pipettes of the pipette head 54. When the pipette head 54 supplies liquids to the measurement cells 17, it accesses the liquid storage sections 61, suctions desired liquids, then moves to the placement space 51 and supplies the liquids to the measuring units 12.

A pipette storage section 63, in which replacement pipettes 62 are stored, is also provided above the frame base 50. The replacement pipettes 62 are interchangeable with the pipettes 19a and 19b. The pipettes 19a and 19b directly contact liquids, and therefore are changed for each liquid that they are utilized with, in order to prevent mixture of different liquids from occurring. Mechanisms for picking up and releasing each of the pipettes 19a and 19b are provided in the pipette head 54, and replacement of the pipettes is performed automatically. During replacement of the pipettes, the pipette head 54 first releases used pipettes 19a and 19b at a disposal section (not shown) . Thereafter. The pipette head 54 accesses the pipette storage section 63 and picks up unused replacement pipettes 62.

A well plate 64, having a plurality of square wells arranged in a matrix, is provided. The wells of the well plate 64 temporarily store liquids, which have been suctioned by the pipettes, or are employed to mix different types of liquids therein, to prepare mixed solutions.

When immobilization is initiated, the frame of the ligand immobilizing device 10 is covered by a cover (not shown), and the interior of the ligand immobilizing device 10 that includes the placement space 51, is shielded from the exterior. The temperature of the interior of the ligand immobilizing device 10 is enabled to be adjusted by a temperature adjusting device (not shown). After supply of ligands to the measurement cells 17, the measuring units 12 are stored on the placement space 51 for a predetermined amount of time, until immobilization of the ligand 21a onto the sensor surfaces 13a is complete. During this time, the ligand solutions 200 within the flow paths 16 are agitated as necessary. The degree of progression of immobilization is influenced by environmental conditions (temperature) of the measuring unit 12. For this reason, the temperature adjusting device maintains the interior of the ligand immobilizing device 10 at a predetermined temperature. The temperature and the amount of storage time is determined appropriately, according to the type of the ligand 21a.

After immobilization of the ligand 21a is complete, a buffer (cleansing liquid) is supplied to the measurement cells 17. The buffer is supplied to the measurement cells 17 by inserting pipettes 19a, which have the buffer suctioned therein, through the slits 43b, while the measurement cells 17 are filled with the ligand solution. When the buffer is expelled from the pipettes 19a through the entrances 16a, the ligand solutions, which fill the flow paths 16, are pushed toward the exits 16b, are discharged therethrough. The ligand solutions, which are discharged through the exits 16b, are suctioned by the pipettes 16b, which perform suctioning operations linked with the supply operations of the pipettes 19a. The liquid within the measurement cells 17 is replaced in this manner.

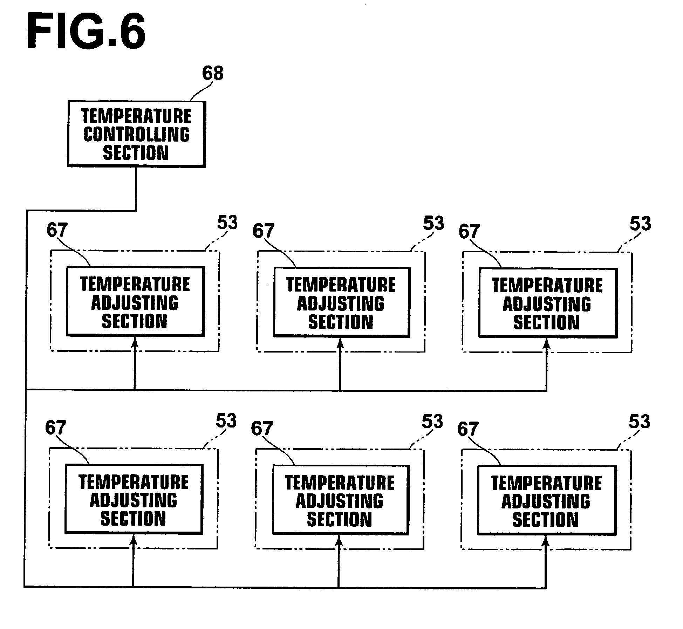

As illustrated in Figure 6, a temperature adjusting section 67, for adjusting the storage temperature of the measuring units 12 housed in each of the holders 52, is provided within each of the bases 53. Each of the temperature adjusting sections 67 constitute a temperature adjusting block. The temperature adjusting sections 67 are heating elements constituted by heating wires, and raise the storage temperature by adding heat energy to the holders 52. A temperature control section 68 individually controls the heat energy generated by each of the temperature adjusting sections 67. The storage temperature of each of the holders 52 is controlled independently in this manner. Accordingly, parallel immobilization processes may be in progress at each of the holders 52, under different immobilization conditions. The temperature control section 68 may control the heating time for each of the holders 52, by controlling the drive time of each temperature adjusting section 67. Although not illustrated in Figure 6, it is preferable for partitions utilizing thermally insulative material to be provided between adjacent bases 53. By dividing the placement stage 51 into sections by the partitions, individual compartments can be formed for each base 53, such that temperatures among the bases 53 do not interfere with each other.

Note that in the present example, heating wires were utilized as the temperature adjusting sections 67, and a description was given regarding a case in which the temperatures of the measuring units 12, which are lower than a target temperature, are raised. Alternatively, in the case that temperatures, which are higher than a target temperature, are to be lowered, a cooling element may be utilized as the temperature adjusting section 67. Of course, both heating elements and cooling elements may be utilized, to enable the temperatures to be adjusted both upward and downward. Pipes, though which water flows, may be employed as the cooling element. Cooling can be performed by providing the pipes within each base 53, and by circulating cooling water therethrough. A Peltier device is an example of a device that can function as both a heating element and a cooling element. As is well known, Peltier devices are elements formed by two types of semiconductors, which are attached to a thin metal plate. The Peltier devices utilize the Peltier effect, in which heat moves from one semiconductor to the other when electrical current is caused to flow therethrough. The Peltier devices have no moving parts, and do not generate noise. Therefore, they are commonly used to cool CPU' s of computers. Heating and cooling can be switched, by changing the directions of the electrical current.

Eight measuring units 12 are housed in each of the holders 52. Therefore, each of the holders 52 may be subject to different temperature conditions A through F. In addition, each of the plurality of measuring units 12 within each of the holders 52 may be subject to different concentration conditions a through f. In this manner, a plurality of measuring units 12, for which immobilization was performed under different temperature conditions and concentration conditions, can be generated within a short amount of time.

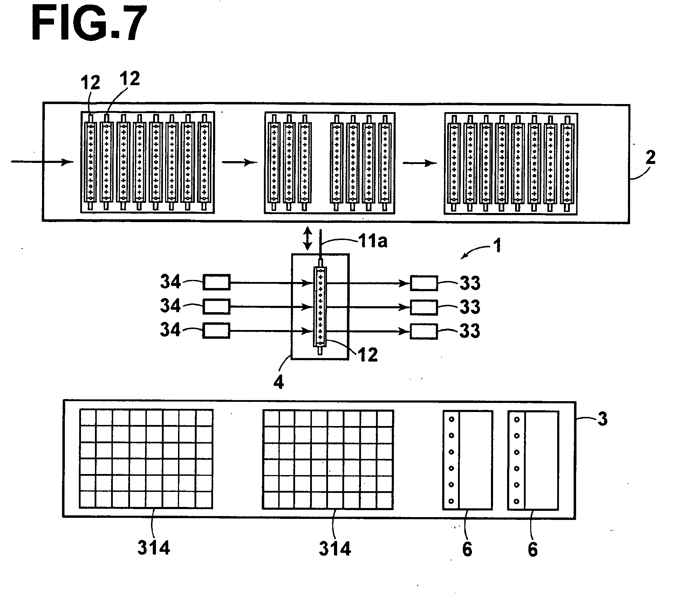

As illustrated in Figure 7, the measuring apparatus 11 is equipped with: the measuring cells 12, which are the three integrally formed measurement cells 17; a surface plasmon sensor section 1, in which a pair of the light source 34 and the detector 33 is provided for each of the measurement cells 17; a constant temperature chamber 2, in which holders 52 that house measuring units 12 therein are stored; a constant temperature chamber 3, in which analyte solution housing plates 314 that house the great number of types of analyte solutions 300 and the liquid storage section 61 that houses the various liquids (cleansing liquid, buffer liquid, and the like) to be supplied to the measurement cells 17 are housed; and a pipette head (not shown) that holds pipette pairs (not shown). The output from each of the detectors 33 is input to the control section 330.

The control section 330 comprises: the bonding data obtaining section 331, for obtaining bonding data regarding ligands and analytes, based on the states of attenuated total reflection output from the detectors 33 of the measuring apparatus 11; and the memory section 332, for recording various types of data therein. The control section 330 controls immobilization operations of ligands and supply operations of analyte solutions to the measurement cells 17, based on protein names and compound names input thereto from an input means (not shown). Ligand data 204 regarding the ligand solutions 200, which are utilized in measurements, analyte data 304 regarding the analyte solutions 300, which are utilized in measurements, and bonding data are correlated and recorded in the memory section 332.

The bonding data obtaining section 331 obtains the attenuated total reflection angle for each measurement, based on the measurement results output from the detectors 33. The bonding data obtaining section 331 calculates variations in the attenuated total reflection angle, which changes with the passage of time. After measurements are completed, the values of bonding speed constants ka; dissociating speed constants kd; dissociating constants KD; and maximum bonding amounts Rmax are obtained as the bonding data, based on variations in the attenuated total reflection angle per unit time (0.5 seconds, for example) . Judgment results regarding whether the values of the bonding speed constants ka; the dissociating speed constants kd; the dissociating constants KD; and the maximum bonding amounts Rmax are within a predetermined range are also obtained, as the bonding data.

Note that the bonding data is displayed at the display section 340, correlated with the protein names and the compound names of the ligands and analytes that were utilized in the measurements.

Hereinafter, the operations of the screening system having the construction described above will be described. An operator inputs a protein name of a ligand and a compound name of an analyte via an input section (not shown), when performing screening. Note that in the case that protein ID' s are recorded in the ligand library 208 as the ligand data 204, the protein ID may be input instead of the protein name. Similarly, in the case that analyte ID's are recorded in the analyte library 308 as the analyte data 304, the analyte ID may be input instead of the compound name.

In the example to be described below, the same predetermined protein (ligand) is immobilized in all of the measurement cells 17, which are to be utilized. A different compound solution (analyte solution) is added to each of the measurement cells 17, and measurements are performed. Thereby, compounds (analytes) that bond with the predetermined protein (ligand) can be screened for. Note that the combinations of the ligands and analytes may be set as desired. As an example, a different protein (ligand) may be immobilized in each measurement cell 17, and the same compound solution (analyte solution) may be supplied. Alternatively, compounds may be employed as the ligands, and proteins may be employed as the analytes.

The control section 330 controls the ligand dispenser 220 to dispense the ligand solution 200, corresponding to the input protein name, from the ligand storage section 202 to the solution housing portions 210 of the ligand solution housing plate 214. Note that the ligand solution 200 is dispensed to the same number of solution housing portions 210 as the number of analyte solutions 300 to be screened. That is, in the case that 1000 types of analyte solutions 300 are to be screened, the ligand solution 200 is dispensed to 1000 solution housing portions 210. At this time, the ligand data 204 that corresponds to the ligand solution 200 to be utilized is read out from the ligand database 206, and recorded in the memory section 332.

Next, the ligand immobilizing device 10 immobilizes the ligand to the measurement cells 17 of the measuring units 12. First, the measuring cells 12 are placed on the placement space 51 of the ligand immobilizing device, while housed in the holders 52. The ligand solution housing plates 214, in which the ligand solution 200 is prepared, is also set on the placement space 51. First, an immobilizing buffer is supplied to each of the measuring cells 17, to moisten the sensor surfaces 13a. Next, activating liquid is supplied to activate the sensor surfaces 13a. After cleansing, the ligand solution 200 is supplied to each of the measurement cells 17 from each of the solution housing portions 210, and immobilization is initiated. In this state, the measuring units 12 are stored on the placement space 51 for a predetermined amount of time. During this time, the ligand 21a within the ligand solution 200 and the linker film 22 bond, and immobilization progresses.

The control section 330 reads out the immobilization temperature and the immobilization time (immobilization protocol) from the ligand data recorded in the memory section 332, and adjusts the temperatures of the holders 52 by controlling the temperature adjusting sections 67 within the bases 53. Note that in the case that a plurality of types of ligands are to be immobilized, a different temperature may be set for each of the temperature adjusting sections 67 (bases 53).

In the case that the ligand is to be immobilized under different concentration conditions for each measuring unit 12, the pipette pairs 19 adjusts ligand solutions having different concentrations at the well plate 64. Then, the plurality of ligand solutions, which have been adjusted to have different concentrations, are supplied to each of the plurality of measuring units 12.

The control section 330 detects the passage of time with a timer (not shown), and issues a immobilization completion command after the immobilization time passes. Blocking processes are administered to each of the measuring units 12 after being cleansed.

In the example described above, the immobilization temperature and the immobilization time are recorded as the immobilization protocol within the ligand data. Alternatively, the immobilization protocol may include ligand concentrations, types of additives, and types of solutions. Immobilization may be performed for each holder 52 while varying these conditions. In addition, each of the plurality of measurement cells within a single measuring unit 12 may be subject to different immobilization conditions.

The measuring units 12, in which immobilization is complete, are moved to the constant temperature chamber 2 of the measuring apparatus 11, in units of holders.

Meanwhile, the analyte solutions are also prepared. The control section 330 controls the analyte dispenser 320 to dispense the analyte solutions 300, corresponding to the input compound names, from the analyte storage section 302 to the solution housing portions 310 of the analyte solution housing plate 314. At this time, the analyte data 304 that correspond to the analyte solutions 200 to be utilized are read out from the analyte database 306, and recorded in the memory section 332. The analyte dispenser 320 reads out barcode data from the bar codes 312 attached to the analyte solution housing plates 312, and outputs the bar code data to the control section 330. The control section records bar code data of the analyte solution housing plate 314 and the row and column number of the solution housing portion 310 that each analyte solution is housed in, correlated with the analyte data thereof. Thereafter, the analyte solution housing plates 312 are moved to the constant temperature chamber 3 of the measuring apparatus 11.

Prior to measurement, a measurement unit 12 is moved from the constant temperature chamber 2 to a measurement position of a unit holding section 4 of the measuring apparatus 11. After the measuring unit 12 is placed on the unit holding section 4, a fixing mechanism (not shown) clamps the protrusions 14b formed on the prism 14, to fix the measuring unit 12 at the measurement position of the unit holding section 4. Thereafter, an analyte solution 300 is suctioned from a solution housing portion 310 of an analyte solution housing plate 314 by a pipette 26a, such as that illustrated in Figure 3. The liquid supplying pipette 26a is inserted through the slits 43b, which cover an entrance 16a, a liquid suctioning pipette 26b is inserted through adjacent slits 43b, which cover an exit 16b, and the analyte solution 300 is supplied to a flow path 16. Measurements are initiated after the analyte solution 300 is supplied to the flow path. Note that when measurements are completed for a measuring unit 12, the measuring unit 12 is moved from the measurement position to the holder 52, a next measuring unit 12 is moved to the measurement position, and measurements are performed thereon.

When measurement results that reflect the states of attenuated total reflection are output from the detectors 33, the bonding data obtaining section 331 first obtains the attenuated total reflection angles, based on the measurement results. Then, variations in the attenuated total reflection angles over time are calculated. After measurements are completed, the values of bonding speed constants ka; dissociating speed constants kd; dissociating constants KD; and maximum bonding amounts Rmax are obtained as the bonding data, based on variations in the attenuated total reflection angles per unit time (0.5 seconds, for example) . Note that at this time, the bonding data can be more accurately obtained, by employing the analyte data, such as the molecular weight of the analyte, recorded in the memory section 332. Judgment results regarding whether the values of the bonding speed constants ka; the dissociating speed constants kd; the dissociating constants KD; and the maximum bonding amounts Rmax are within a predetermined range are also obtained, as the bonding data.

Note that the bonding data is output to the display section 340, correlated with the ligand name and the analyte name. The display section 340 displays the data.

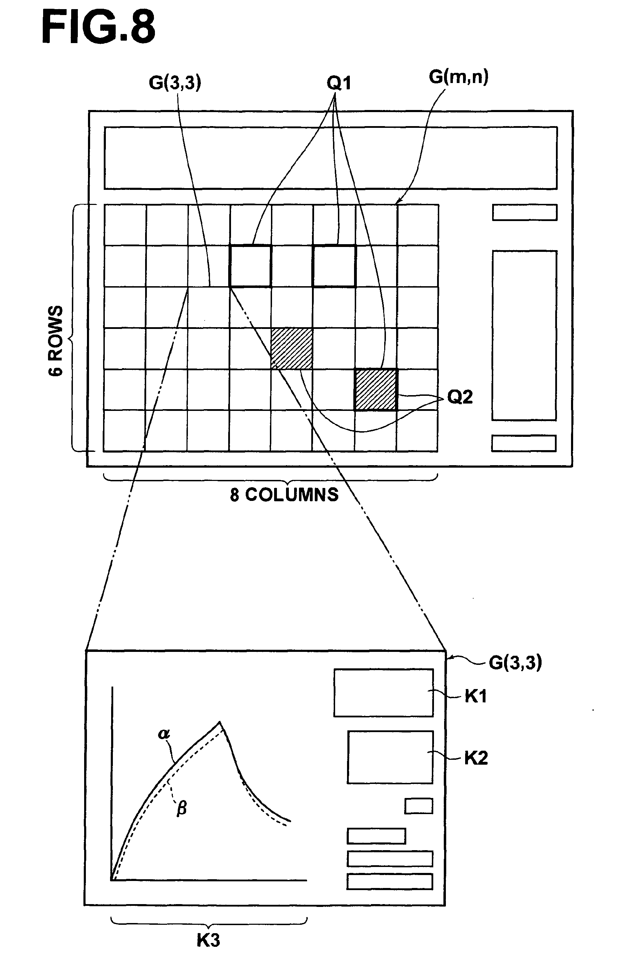

Figure 8 illustrates the bonding data, which is displayed at the display section 340. Each of the 48 thumbnail images G(m, n), arranged in six rows and eight columns, illustrated in Figure 8 is displayed corresponding to the housing position F(m, n) of each analyte solution 300 in the analyte solution housing plate 314.

That is, the display section 340 displays the analyte data corresponding to each of the housing positions F(m, n) correlated with the bonding data regarding the analyte solution 300 housed in each of the housing positions F(m, n) . The bonding data are represented by each of the thumbnail images G(m, n), which are displayed corresponding to the housing positions F(m, n).

Thumbnail images G, of which the maximum bonding amount Rmax is within the predetermined range, are displayed bordered by bold lines Q1. In the case that the maximum bonding amount Rmax is not within the predetermined range, the border of the thumbnail images G are not changed. Thumbnail images G, of which the dissociating speed constant kd is within the predetermined range, are displayed with a colored background Q2. In the case that the dissociating speed constant kd is not within the predetermined range, the background of the thumbnail images G are not changed. Similarly, if the manner of display of the thumbnail images G is changed according to whether the bonding speed constant ka, the dissociating constant KD and bulk correction are within their predetermined ranges, the bonding data can be easily understood. Examples of different manners of display are: different colors, blinking display, presence/absence of hatching, and different border thicknesses.

Further, if a single thumbnail image G from among the thumbnail images G (m, n), for example, thumbnail image G (3, 3), is clicked, an enlarged image G is displayed, as illustrated in Figure 8. The following items are displayed in the enlarged image G: (1) the ligand name and the analyte name of the ligand and the analyte employed in the measurement (indicated by K1 in the figure); (2) kinetic values (indicated by K2 in the figure); and (3) a sensorgram (indicated by K3 in the figure). Note that the bonding speed constant ka, the dissociating speed constant kd, the dissociating constant KD, and the maximum bonding amount Rmax are collectively referred to as kinetic values.

The sensorgram is a graph that illustrates changes in the attenuated total reflection angle over time, with time as its horizontal axis and angles as its vertical axis. A bonding/dissociating curve α, based on actual measurements, is drawn as a solid line, and an estimated bonding/dissociating curve β, based on the kinetic values, is drawn as a broken line. Note that the maximum value of the horizontal axis is automatically determined, based on the amount of time required for measurement. The scale of the vertical axis that indicates angles can be set by the operator.

The control section 330 records the obtained bonding data within the analyte database 304 of the analyte library 308. By recording the bonding data in the analyte database 304, the bonding data may be employed as a candidate selecting condition, in the case that focused screening, in which the number of analytes to be screened is narrowed by certain conditions, is to be performed. Thereby, the hit rate of the analytes can be improved.

As is clear from the above description, in the screening system of the present invention, the ligand immobilizing device 10 supplies the ligand solution 200 to the sensor surface 13a of the measurement cell 17, prior to the measuring apparatus 11 performing measurement, and the measurement cell 17 is held while the ligand contained in the ligand solution is being immobilized on the sensor surface 13a. Therefore, measurement by the measuring apparatus 11 and the ligand immobilizing process by the ligand immobilizing device 10 can be performed in parallel, improving efficiency in immobilization of ligands and obtainment of bonding data. Accordingly, bonding data regarding a great number of combinations of ligands and analytes are enabled to be obtained within a short period of time. Further, the specified analyte solution 300 is removed from the analyte solution storage section 302 and supplied to the measuring apparatus 11 prior to measurement. After measurement, the analyte data of the specified analyte solution is recorded and displayed, correlated with the bonding data obtained by the bonding data obtaining section 331. Thereby, the correspondence among obtained bonding data and analytes can be managed automatically.

The bonding data obtaining section 331 obtains the bonding data regarding the ligand and the analyte, based on the measurement results of the measuring apparatus 11 and the analyte data, such as the molecular weight of the analyte. Therefore, more accurate bonding data is enabled to be obtained.

The analyte dispenser 320 removes a specified analyte solution 300 from the analyte storage section 302 and supplies the specified analyte solution 300 to a solution housing portion 310 of a solution housing plate 314, on which a bar code 312 is attached. The memory means records the bar code data of the solution housing plate 314, the position of the solution housing portion 310, and the analyte data 304 regarding the specified analyte solution 300, correlated to each other. Therefore, erroneously selecting an analyte, which is not the specified analyte, can be prevented, thereby improving the reliability of screening.

During the ligand immobilizing process, a specified ligand solution 200 is removed from the ligand storage section 202 of the ligand library 208 and supplied to the ligand immobilizing device 10. The memory section 332 records the ligand data 204 of the specified ligand solution 200, correlated with the bonding data obtained by the bonding data obtaining section 331. The display section 340 displays the ligand data 204 of the specified ligand solution 200, correlated with the bonding data obtained by the bonding data obtaining section 331. Therefore, the correspondence among the obtained bonding data and ligands can be managed automatically.

The specified ligand solution 200 is removed from the ligand storage section 202 and supplied to a solution housing portion 210 of a solution housing plate 214, on which the bar code 212 is attached. The memory means records the bar code data of the solution housing plate 214, the position of the solution housing portion 210, and the ligand data 204 regarding the specified ligand solution 200, correlated to each other. Therefore, erroneously selecting a ligand, which is not the specified ligand, can be prevented, thereby improving the reliability of screening.

Note that in the present embodiment, the ligand data is recorded in the ligand database 206, and the analyte data is recorded in the analyte database 306. Alternatively, the ligand data and the analyte data may be recorded in a single integrated database, along with all of the data related to the screening process. In this case, the memory section 332 may be the integrated database.

Note also that in the above example, the surface plasmon sensor was described as the measuring apparatus that utilizes attenuated total reflection, and the measuring unit comprising the plurality of measurement cells was employed therein. However, the structure of the measuring unit is not limited to such a construction. The measuring unit may be that which comprises a single measurement cell, or that which comprises a great number of measurement cells, arranged two dimensionally.

In addition, the measuring unit described in the above example was that in which the metal film, the flow path, and the prism are integrated. However, the measuring unit is not limited to such a construction. A configuration may be adopted, wherein the prism or the flow path is structured as a separate component. As an example, a measurement chip, comprising a metal film having a sensor surface formed on a glass substrate; a prism; and a flow path may be combined.

The placement space of the ligand immobilizing device 10 was described as that in which the plurality of bases are arranged. Alternatively, the placement space may be constituted by a plurality of placement chambers, which are spatially partitioned by partition walls or the like, and which are capable of housing each measuring unit separately. If this configuration is adopted, changing the temperature within each placement chamber is enabled.

Further, in the above embodiment, the ligand immobilizing device was described as being a component of the screening system separate from the measuring apparatus. Alternatively, the ligand immobilizing device may be built in to the measuring apparatus, and integrated therewith.

Note that in the present embodiment, a case was described in which the surface plasmon sensor that causes surface plasmon to be generated on the sensor surface and detects attenuation in reflected light was utilized as the measuring apparatus that utilizes attenuated total reflection. However, the measuring apparatus that utilizes attenuated total reflection is not limited to being the surface plasmon sensor, and other types of sensors that utilize attenuated total reflection may be employed. As an example of another type of sensor that utilizes attenuated total reflection, a leaky mode sensor is known. The leaky mode sensor comprises: a dielectric block; and a thin film layer constituted by a cladding layer and an optical waveguide layer, formed on the dielectric block. One surface of the thin film layer is a sensor surface, and the other surface is a light incident surface. When light enters the light incident surface such that total internal reflection conditions are satisfied, a portion of the light passes through the cladding layer and is absorbed into the optical waveguide layer. If surface acoustic waves are generated in the optical waveguide layer, the light reflected at the light incident surface is greatly attenuated. The incident angle at which the surface acoustic waves are generated changes according to the refractive index of a substance on the sensor surface, in a manner similar to the resonance angle for surface plasmon. By detecting the attenuation in the reflected light, reactions on the sensor surface can be measured.

## Claims

1. A screening system, for performing measurements regarding combinations of pluralities of types of ligands and analytes, and for obtaining bonding data for each combination of ligands and analytes, comprising:
a measuring apparatus (11) that utilizes attenuated total reflection, comprising:
a measurement cell (17), including: a dielectric block (14); a thin film layer (13), which is formed on a surface of the dielectric block (14) and on which a ligand is immobilized; and an analyte holding mechanism (41), for holding an analyte on the thin film layer (13); and
a measuring section (31), for causing a light beam to enter the dielectric block (14) of the measurement cell (17), which is at a measurement position, at various angles of incidence, such that conditions for total internal reflection are obtained at an interface between the dielectric block (14) and the thin film layer (13), and for measuring the state of attenuated total reflection of the light beam, which is totally internally reflected at the interface; and
bonding data obtaining means (331), for obtaining bonding data regarding the ligand and the analyte, based on the measurement results obtained by the measuring apparatus (11); **characterized by** further comprising:
ligand immobilizing means (10), for supplying a ligand to the thin film layer (13) of the measurement cell (17) at a position different from the measurement position prior to the measuring apparatus (11) performing measurement, and for holding the thin film layer (13) while the ligand is being immobilized on the thin film layer (13);
an analyte library (308), equipped with an analyte database (306) for recording storage sections (302), at which each of the plurality of types of analytes is stored, correlated with analyte data for each type of analyte;
analyte supplying means (320, 314), for removing a specified analyte from an analyte storage section (302) and for supplying the specified analyte to the measuring apparatus (11) ;
memory means (332) for recording the analyte data of the specified analyte, correlated with the bonding data obtained by the bonding data obtaining means (331); and
display means (340), for displaying the analyte data of the specified analyte read out from the memory means (332), correlated with the bonding data obtained by the bonding data obtaining means (331).

2. A screening system as defined in Claim 1, wherein:
the bonding data obtaining means (331) obtains the bonding data regarding the ligand and the analyte, based on the measurement results of the measuring apparatus (11) and the analyte data.

3. A screening system as defined in either of Claim 1 and Claim 2, wherein:
the analyte database (306) records the bonding data regarding the ligand and the analyte obtained by the bonding data obtaining means (331), correlated with the analyte data of the analyte.

4. A screening system as defined in any one of Claim 1, Claim 2, or Claim 3, wherein:
the analyte supplying means (320, 314) comprises: a plurality of solution housing plates (314), which have ID' s, and in which a plurality of solution housing portions (310) are arranged two dimensionally; and analyte dispensing means (320), for removing the specified analyte from the analyte storage section (302) and supplying the specified analyte to a solution housing portion (310) of a solution housing plate (314); and
the memory means (332) records the ID of the solution housing plate (314), the position of the solution housing portion (310), and the analyte data regarding the specified analyte, correlated to each other.

5. A screening system as defined in any one of Claim 1, Claim 2, Claim 3, or Claim 4, further comprising:
a ligand library (208), equipped with a ligand database (206) for recording storage sections (202), at which each of the plurality of types of ligands is stored, correlated with ligand data for each type of ligand; and
ligand supplying means (214, 220), for removing a specified ligand from a ligand storage section (202) and for supplying the specified ligand to the ligand immobilizing means (10); wherein:
the memory means (332) records the ligand data of the specified ligand, correlated with the bonding data obtained by the bonding data obtaining means (331); and
the display means (340) displays the ligand data of the specified ligand read out from the memory means (332), correlated with the bonding data obtained by the bonding data obtaining means (331).

6. A screening system as defined in Claim 5, wherein:
the ligand supplying means (214, 220) comprises: a plurality of solution housing plates (214), which have ID's, and in which a plurality of solution housing portions (210) are arranged two dimensionally; and ligand dispensing means (220), for removing the specified ligand from the ligand storage section and supplying the specified ligand to a solution housing portion (210) of a solution housing plate (214); and
the memory means (332) records the ID of the solution housing plate (214), the position of the solution housing portion (210), and the ligand data regarding the specified ligand, correlated to each other.
